# EUROPEAN PATENT APPLICATION

(11) **EP 4 552 571 A1**
(43) Date of publication of application: **14.05.2025**
(21) Application number: 23208552.2
(22) Date of filing: 08.11.2023
(51) Int. Cl.: A61B 5/20, A61B 5/00, A61M 25/00

(54) **URINARY PROBE ASSEMBLY**

(71) Applicant: Wellspect AB, 431 21 Mölndal (SE)
(72) Inventor: LOVMAR, Martin, 431 69 Mölndal (SE); UTAS, Jan, 434 95 Kungsbacka (SE); D´ESPINDULA, Gabriel, 418 75 Göteborg (SE)
(74) Representative: Aldridge, Henry Alexander

(57) **Abstract**

The present disclosure relates to a urinary probe assembly comprising a shaft wire and a sensor unit, arranged at a proximal end of the shaft wire, wherein the sensor unit comprises at least one sensor configured to measure a property of a urinary bladder and/or a fluid inside the urinary bladder. The urinary probe assembly further comprises an introducer device including a splitable tubular sheath, wherein the introducer device is arranged to accommodate at least a part of the shaft wire, and wherein at least a part of an outer surface of the tubular sheath is provided with a hydrophilic coating/surface.

## Description

### TECHNICAL FIELD OF THE INVENTION

The present invention relates to a urinary probe assembly comprising a urinary probe and an introducer device.

### BACKGROUND OF THE INVENTION

Different types of catheters are known to be inserted into bodily openings of humans and other mammals. Urinary catheters are for example commonly used for insertion into the urethra for drainage of urine. Some urinary catheters are intended for short time use, such as intermittent urinary catheters, intended to be inserted for a short period of time, such as a few minutes, and then be withdrawn again. Other catheters are intended to be used for longer time periods, such as Foley catheters, intended to be inserted for hours, days or even weeks.

In cystometry, the function of the bladder is evaluated by measuring the pressure inside the bladder when voiding. Since the process may be uncomfortable for the patient, and/or the patient may be required to be still, the bladder is often filled artificially so that voiding can commence after only a short time.

A cystometric measurement is typically achieved with a double catheter procedure wherein two catheters are introduced in the bladder via the urethra. The first catheter is equipped with a pressure sensor and the second catheter comprises a lumen for injecting a liquid (such as water) into the bladder. Liquid is injected into the bladder via the second catheter until the patient feels the urge to void, whereby the patient voids the bladder and the pressure in the bladder during the voiding process is measured using the pressure sensor of the first catheter. Since the bladder is filled artificially, the entire process can be rather quick, taking from 15 minutes up to at most 60 minutes to complete.

Alternatively, the cystometric measurement is performed with a single catheter procedure where a specifically adapted catheter, featuring both a lumen for introduction of fluid into the bladder and a pressure sensor, is used instead of two catheters.

A drawback with existing devices and procedures for cystometric measurements is that the catheters involved are uncomfortable to use and the equipment necessary for injecting liquid to artificially fill the bladder is expensive, complicated and bulky. To perform cystometric measurements patients are often required to travel to a hospital due to the specialized equipment needed. In some procedures, two catheters are used simultaneously, which can cause additional discomfort for the patient.

To increase comfort for the patient the process is performed quickly, by artificially filling the bladder allowing voiding to occur soon after starting the procedure. However, there are some risks involved with artificially filling the bladder and recommendations putting a limit on how quickly the bladder can be filled by fluid injection, meaning that it may not be possible to shorten the duration of the procedure by injecting fluid more rapidly. Also, it is envisaged that the pressure profile measured when voiding after filling the bladder artificially, while there is one or more comparatively stiff catheters extending through the urethra causing discomfort, does not completely correspond to the pressure profile of a regular voiding process, without artificial bladder filling or catheters extending through the urethra. This means that there may be a comparatively large level of uncertainty surrounding this type of measurement.

In view of these drawbacks, there is a need for a new and improved device for performing cystometric measurements.

### SUMMARY OF THE INVENTION

It is a purpose of the present invention to overcome at least some of the drawbacks associated with existing solutions for performing cystometric measurements.

According to a first aspect of the invention there is provided a urinary probe assembly comprising a shaft wire and a sensor unit arranged at a proximal end of the shaft wire, wherein the sensor unit comprises at least one sensor configured to measure a property of a urinary bladder and/or a fluid inside the urinary bladder. The urinary probe assembly further comprising an introducer device including a splitable tubular sheath, wherein the introducer device is arranged to accommodate at least a part of the shaft wire, and wherein at least a part of an outer surface of the tubular sheath is provided with a hydrophilic coating/surface.

The shaft wire and the sensor unit arranged on the shaft wire are collectively referred to as a "urinary probe". Accordingly, the urinary probe assembly may be said to comprise the urinary probe and the introducer device. The shaft wire of the urinary probe can be made very thin and soft allowing it to comfortably remain in the urinary bladder for long periods of time after insertion. For example, the urinary probe can remain in the bladder with the shaft wire extending through the urethra for at least one hour, several hours, one day, several days, one week or several weeks. By comparison, traditional catheters used for cystometric measurements comprise at least one lumen and are thus necessarily much wider and/or stiffer compared to the envisaged urinary probe of the present invention. In some implementations, the shaft wire does not comprise a lumen (or any void space) and is shaped like a thin thread. By being devoid of any lumen, the shaft wire can be made even thinner and softer compared to traditional catheters used for cystometric measurements. In some implementations, the shaft wire comprises (e.g. comprises only) a communication element (e.g. an electrically conducting wire or an optical fiber) coated with one or more coating layers. Alternatively, the shaft does not comprise any transmission element and data transmission to/from the sensor(s) is performed wirelessly.

On its own, the thin and soft urinary probe may be difficult, or even impossible, to insert into the bladder via the urethra. To this end, at least a portion of the urinary probe is covered with a removable introducing device which adds rigidity. The introducing device is further covered with a hydrophilic material that, when wetted, becomes slippery and exhibits low friction, thereby making it comfortable and easy to introduce via the urethra. At the same time, the hydrophilic material makes the introducing device easy to remove from the urethra after it has served its purpose of guiding the urinary probe to the urinary bladder.

There are many types of hydrophilic coatings or layers that can be used on the introduced device. Preferably, the hydrophilic coating/surface comprises polyvinylpyrrolidone (PVP). Even though PVP is the preferred hydrophilic material, other hydrophilic materials may be used, such as hydrophilic polymers selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, hydroxy propyl methyl cellulose, methyl cellulose, copolymer of vinylpyrrolidone and hydroxy ethylmethyl acrylate or copolymer of polymethylvinyl ether and maleinic acid any hydride. Further, hydrophilic coating/surface is to be understood in a broad sense, and in embodiments the hydrophilic coating/surface may comprise an entire tubular sheath formed of a hydrophilic material.

With the urinary probe arrangement of the present invention, a completely new type of long-term cystometric measurements can be performed. Since the urinary probe is much thinner and softer compared to conventional catheters used during conventional cystometric measurements, the urinary probe can comfortably remain in place for many hours, the majority of a day or even several days with increased comfort. This allows the bladder to fill naturally and the patient to void naturally with the urinary probe still in place. For example, it is envisaged that the urinary probe remains in place during multiple natural, bladder filling-voiding cycles allowing the property of the urinary bladder or fluid therein to be measured for a long period of time. That is, the urinary probe arrangement of the present invention may enable long term detection of properties (e.g. pressure, temperature, or urine constituent concentration) during many hours, days or even weeks which allows the urinary probe to detect potential anomalies that only occur rarely, e.g. during a certain time of the day, a certain phase of the urinary bladder cycle or when the user/patient performs certain activities (e.g. sleeping or walking). These types of long term measurements are not possible using traditional cystometric catheters, which can only be used for much shorter measurements while requiring the patient to be still or keeps the patient awake.

Additionally, when measurement is performed with the thin and soft urinary probe during voiding, the urinary probe is much less obstructive compared to traditional catheters, allowing the voiding process to more closely resemble a natural voiding process with no foreign object present in the urethra.

While the urinary probe arrangement enables a completely new type of catheter-free and long term cystometric measurements it is understood that the urinary probe assembly can also be used together with regular urinary catheters by users who void using catheterization. For example, to empty the bladder when the urinary probe is in place a user may introduce a urinary catheter alongside the urinary probe and empty the bladder via the urinary catheter.

In some implementations, the sensor unit comprises at least one pressure sensor configured to measure a fluid pressure. The term "fluid" is interpreted broadly so as to cover both gases and liquids. Hereby it is understood that the pressures sensor may be configured to measure a liquid pressure, a gas pressure or both.

The pressure sensor may be any type of pressure sensor suitable for measuring the pressure in the urinary bladder over time. The urinary probe may then be used to determine a pressure-time diagram illustrating how the pressure changes over time in the urinary bladder, such as during voiding, (natural) filling of the bladder, or when the user performs an activity, such as sleeping or walking. A pressure-time diagram can then be analyzed by the user or a medical professional to determine any deviations that may indicate that further investigations are to be performed.

In some implementations, the pressure sensor is encased within a fluid filled cavity.

As an example, the cavity may be at least partially formed by a membrane. The cavity protects the pressure sensor and when the cavity is filled with gas this allows e.g. a gas pressure sensor or pressure sensor to be used, which otherwise would be damaged by the urine or which is incapable of measuring a liquid pressure. Alternatively, the cavity can be filled with a liquid, whereby a liquid pressure sensor is used. This allows e.g. a liquid pressure sensor which would otherwise be damaged by the urine to be used if the liquid inside the cavity is compatible with the liquid pressure sensor. For example, the liquid inside the liquid filled cavity is water or distilled water.

The pressure inside the urinary bladder will influence the fluid pressure inside the cavity whereby the pressure sensor, while not being in direct contact with the space inside the urinary bladder, is still able to measure the pressure inside the bladder. For example, if a gas pressure sensor is used inside a gas filled cavity, the pressure inside the bladder can be measured indirectly by measuring the gas pressure in the cavity, since the gas pressure in the cavity will be influenced by the pressure inside the urinary bladder.

In some implementations, the sensor unit comprises at least one sensor configured to measure at least one of: temperature, pH and salinity in addition to, or as an alternative to, the pressure sensor.

Accordingly, the urinary probe can be used to measure a large variety of physical or chemical parameters inside the urinary bladder. Additionally, it is understood that one or more different sensors can be arranged in the sensor unit such that one or more physical or chemical parameters can be measured. Some sensor(s), such as a salinity sensor, are arranged to be in direct contact with the inside of the urinary bladder or the urine therein when the urinary probe is put in place whereas other sensors, such as a pressure sensor or temperature sensor, may be arranged in a cavity or optionally in direct contact with the inside of the urinary bladder or the urine therein.

In some implementations, an outer diameter of the shaft wire is less than less than 3 mm, less than 2 mm, less than 1.5 mm, less than 1 mm, less than 0.75 mm or less than 0.5 mm. For example, the outer diameter of the shaft wire is in the range of 2 mm to 0.5 mm, such as between 1.5 mm and 0.5 mm or between 1 mm and 0.5 mm.

That is, the shaft wire is made very thin, and much thinner than the diameter of typical urinary catheter which is typically CH14 for men and CH10 for women. The abbreviation "CH" stands for Charriere and one CH corresponds to a 1/3 millimeter diameter or, conversely, the CH unit divided by three indicates the diameter in millimeter (i.e. the diameter in millimeter multiplied by three corresponds so to the Charriére size of the catheter).

Since the shaft wire preferably is devoid of any lumen or hollow space it can be made much thinner and softer than a traditional urinary catheters used for cystometric measurements and therefore be more comfortable when the shaft wire extends through the urethra into the bladder. Traditional catheters used of cystometric measurements are by necessity rather stiff so as to enable insertion through the urethra without bending or kinking and so as to not collapse while being inside the urethra which would prevent injection of liquid through the lumen.

With the urinary probe assembly of the present invention the introducer device alleviates the need for the shaft wire to be stiff to allow insertion without kinking and the as the shaft wire may be devoid of a lumen for fluid injection there is no need for the shaft wire to be stiff to avoid collapsing.

Additionally, since the shaft wire is thin and soft, it further does not obstruct urine being voided through the urethra which enables the voiding process to more closely resemble a natural voiding process which in turn may facilitate more accurate measurements.

The above shaft wire diameters are merely exemplary, and it is envisaged that an even thinner shaft wire, having a diameter less than 1 mm, less than 0.66 mm or even less than 0.33 mm can be used. On the other hand, the shaft wire can also be much thicker provided it still is soft and comfortable, for example the shaft wire may have a diameter which is smaller than 4 mm, smaller than 3.3 mm or smaller than 2.66 mm.

In some implementations, the introducer device is arranged to accommodate at least an insertable part of the shaft wire.

The introducer device will hereby provide rigidity to a part of the shaft wire which will enter into the urethra during use. Preferably, the introducer device accommodates the entire insertable part of the shaft wire. As will be described below, the sensor unit is optionally also accommodated, or partially accommodated, by the introducer device. It is also envisaged that the introducer does not cover the sensor unit.

In some implementations, the introducer device comprises a first end and a second end remote from the first end, and at least one tear line extending between the first and second ends.

The tear line may e.g. be a line forming a weakening in the material, such as a line of perforations, a line made of a different, weaker material, a line along which the wall thickness is thinner, e.g. by being partly cut through, etc. In an embodiment, the cut through or perforations extend over more than 50% of the wall thickness of the tubular sheath, and preferably more than 70%, such as 80-90%. When a force is applied on the introducer device, the tear line will preferably be the part of the introducer device that is separated, thereby forming a split. The splitting enables the removal of the introducer device from the urinary probe when it has been inserted into the urethra.

In some implementations, the introducer device further comprises at least one gripping element arranged at, or in the vicinity of, a non-insertable end of the introducer device.

The gripping element may e.g. be in the form of handles or flanges protruding out from the tubular sheath. In an embodiment, the gripping element may protrude outwardly, at least partially in a radial, lateral direction. In an embodiment, at least two gripping elements are provided, preferably arranged separated or distributed along the circumference of the tubular sheath, and preferably at opposite sides, and protruding in opposite directions. However, only one gripping element may also be used, or more than two gripping elements.

In some implementations, the tubular sheath has a bending stiffness which is greater than the bending stiffness of the shaft wire. For example, the bending stiffness of the tubular sheath is at least 20% greater than the bending stiffness of the shaft wire or, preferably, the bending stiffness of the tubular sheath is at least 50% greater than the bending stiffness of the shaft wire.

That is, the introducer device, having the greater bending stiffness will add rigidity to the shaft wire, allowing the shaft wire to have a lower bending stiffness which enhances comfort when the urinary probe is inserted into the urinary bladder and the shaft wire extends through the urethra. The bending stiffness of the introducer device may be defined as the bending stiffness of the introducer device when it is arranged around the urinary probe.

In some implementations, the sensor unit or shaft wire comprises an expandable retention element, and wherein the introducer device is arranged to maintain the expandable retention element in a compressed state when accommodating the shaft wire.

The introducer device is preferably arranged to maintain the expandable retention element in a compressed state when accommodating the urinary probe. Hereby, the retention element is compressed by the introducer device, for easy and simple insertion into the urethra. When the introducer device is subsequently removed, the retention element is allowed to expand, and will then serve to maintain the urinary probe in place.

In some implementations, the shaft wire comprises a signal transmission element configured to enable communication between a distal end connector of the shaft wire and the at least one sensor of the sensor unit.

With a signal transmission element, measured data can be conveyed from the sensor(s) of the sensor unit to the distal end connector and/or control signals and/or power can be conveyed to the sensor(s) of the sensor unit via the distal end connector. The signal transmission element may e.g. be an electrically conductive wire for electrical communication or an optic waveguide (such as an optical fiber) for optical communication.

According to a second aspect of the invention there is provided a urinary probe system comprising the urinary probe assembly according to the first aspect, and an external device configured to be connected to the distal end connector of the urinary probe.

The external device may comprise a power source and controller for powering and controlling the sensor(s) of the sensor unit. Thereby it is not necessary to integrate a power source or controller into the urinary probe, enabling the urinary probe to be made very small and thin. In some implementations, the urinary probe assembly is intended for single time use and to be disposed after a cystometric measurement has been performed. On the other hand, the external device is preferably intended for multiple time use and to be connected to a new urinary probe after a previous urinary probe has been used and disposed of.

### BRIEF DESCRIPTION OF THE DRAWINGS

The present invention will be described in more detail with reference to the appended drawings, showing currently preferred embodiments of the invention.
Figure 1 illustrates a side view of a urinary probe according to some implementations.
Figure 2 shows a cross-sectional side view of a urinary probe assembly according to some implementations.
Figure 3 shows a side view of an introducer device according to some implementations.
Figure 4A-D shows a cross-sectional view of the tubular sheath with different number of tear lines according to some implementations.
Figure 5A and 5B show a cross-sectional and side view, respectively, of the details of the urinary probe assembly comprising a guide arrangement according to some implementations.
Figure 6 illustrates how the introducer device is opened to release the urinary probe according to some implementations.
Figure 7A-C show how the tubular sheath extends to a proximal portion of the urinary probe according to different embodiments.
Figure 8A-C show different examples of retention features which may optionally be provided on the urinary probe.
Figure 9 depicts a urinary probe assembly comprising a package with a wetting fluid according to some implementations.
Figure 10 shows schematically an external device configured to be connected to the urinary probe according to some implementations.

### DETAILED DESCRIPTION OF CURRENTLY PREFERRED EMBODIMENTS

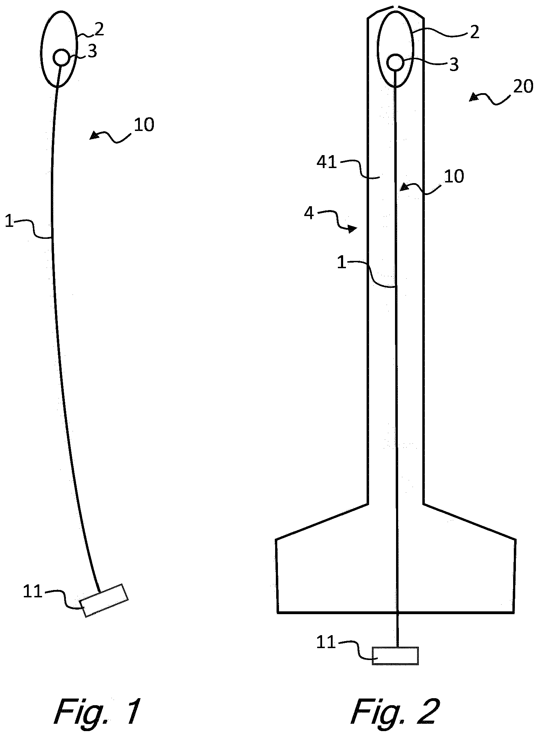

Fig. 1 schematically depicts a urinary probe 10. The urinary probe 10 comprises a shaft wire 1 with a sensor unit 2 attached to one end of the shaft wire 1. The shaft wire 1 extends from a proximal end to a distal end wherein the sensor unit 2 is arranged at the proximal end of the shaft wire 1. When in use, the shaft wire 1 is inserted (using an introducer device) into the urethra of a patient with the proximal end first. That is, the sensor unit 2 arranged at the proximal end of the shaft wire 1 enters into the urethra first, followed by the shaft wire 1. When at least the sensor unit 2 (and optionally also a proximal portion of the shaft wire) has passed into the urinary bladder, the shaft wire 1 extends through the urethra such that a distal portion of the shaft wire 1 remains outside the urethra. That is, when the urinary probe is in use, the proximal direction is pointing generally towards the user/patient and the distal direction is the opposite direction, pointing generally away from the user/patient.

The length of shaft wire 1 is thus configured such that when the sensor unit 2, and optionally also a proximal portion of the shaft wire 1, has entered the urinary bladder through the urethra a distal portion of the shaft wire 1 remains outside the urethra. To this end, the length of the shaft wire 1 may be adapted for male or female patients where the shaft wire 1 is generally longer when adapted for use on male patients. For example, the shaft wire 1 is in the range 80-140 millimeters for a female user and in the range 200-350 millimeter for a male user. In some implementations, the shaft wire 1 is preferably even longer such that the sensor unit 2 can remain in the urinary bladder even when the distal end of the shaft wire is connected to an external device which may be held towards the inside of the thigh of the user, as will be described in the below.

The shaft wire 1 may also comprise an insertion stopper, such as a collar, on a distal portion of the shaft wire 1 that prohibits the shaft wire 1 from entering too far into the bladder. Accordingly, the shaft wire 1 comprises two parts, an insertable part, comprising the sensor unit 2 and a proximal portion of the shaft wire 1, and a non-insertable part comprising a distal portion of the shaft wire 1.

The sensor unit 2 comprises at least one sensor 3 configured to measure a property of the urinary bladder and/or a fluid inside the urinary bladder. For example, the at least one sensor 3 is configured to measure the pressure inside the urinary bladder. Additionally or alternatively, the at least one sensor 3 is configured to measure at least one of the temperature inside the bladder, the pH level inside the bladder and the salinity inside the bladder.

In some implementations, the sensor unit 2 comprises more than one sensor 3 with each sensor 3 being configured to perform individual measurements. Each sensor 3 may measure different properties (e.g. one sensor measures pressure and another sensor measures temperature) but it is also envisaged that two sensors may measure the same property (e.g. at least two sensors measure pressure) whereby the at least two measurements of the same property can be averaged or otherwise combined to improve measurement accuracy.

The sensor unit 2 may comprise additional electronic components in addition to the sensor (3) itself, such as a controller. Preferably, however, the sensor unit 2 comprises as few electronic components as possible besides the sensitive element of the sensor 3, to keep the sensor unit 2 as small and compact as possible which increases comfort for the user when the sensor unit 2 is introduced into the bladder via the urethra and when the sensor unit 2 remains in the urinary bladder. In some implementations, each sensor 3 may be electrically and/or optically connected to an external device via a communication element (e.g. an electrically conductive wire or optical fiber) integrated in the shaft wire 1 meaning that many electrical components for controlling and powering each sensor 3 can be provided in the external device.

Alternatively, the sensor module 2 may comprise a wireless transmitter, and optionally a wireless receiver, for wirelessly transmitting sensor data to an external device, and optionally receiving control data from an external device. Power may also be supplied wirelessly, e.g. using inductive coupling to power the sensor(s) 3 of the sensor unit 2. Alternatively, a power source such as a battery or fuel-cell (e.g. a biofuel-cell) may be integrated in the sensor unit 2 whereby no communication element is needed in the shaft wire 1. The primary purpose of the shaft wire 1 then becomes to allow easy withdrawal of the urinary probe 10 from the bladder.

In some embodiments, the sensor unit 2 has a larger diameter compared to the outer diameter of the shaft wire 1. For example, the sensor unit 2 has a diameter which is at least two times the diameter of the shaft wire 1, at least three times the diameter of the shaft wire 1 or at least four times the diameter of the shaft wire 1. A larger sensor unit 2 may facilitate integration of additional sensors 3 and/or more accurate sensors 3. However, in some embodiments the sensor unit 2 is very small, having a diameter which is equal to or even smaller than the diameter of the shaft wire 1.

The shaft wire 1 is preferably realized with a very small diameter being much smaller than the diameter of a conventional urinary catheter. For example, the outer diameter of the shaft wire 1 is less than 4 mm, less than 2.66 mm or even less than 2 mm. In some implementations, the outer diameter of the shaft wire 1 is less than 1.66 mm, 1.5 mm, 1 mm, 0.5 mm, 0.33 mm or even less than 0.16 mm. In some implementations, the shaft wire 1 has a diameter which is at least 0.1 mm such as at least 0.15 mm or at least 0.2 mm.

The shaft wire 1 of the urinary probe 10 may comprise a communication element such as a wire or optical fiber that allows communication with sensor(s) 3 of the sensor unit 2. Electrical power can also be supplied to the sensor(s) 3 via the communication element when it is realized as an electrical conductor. Since the sensor(s) 3 in the sensor unit 2 can be realized with low power, low voltage, sensor(s) 3, the voltages and power transmitted over the electrical wire will be very small allowing a thin non-conductive cover layer to protect and electrically isolate the electrical wire to be used, whereby the shaft wire 1 can be made very thin. The communication element is terminated in a connector 11, allowing the communication element to communicate with an externa device via the connector 11. The connector 11 may also act as an insertion stopper, prohibiting the urinary probe 10 from being inserted too far into the urethra and urinary bladder.

The shaft wire 1 and/or sensor unit 2 may comprise an outer layer or coating of one or more plastic materials, and in particular polymer and/or thermoplastic materials, such as materials conventionally known for use as catheter shafts and the like. The urinary probe 10 may e.g. mainly be formed by polyurethane, nylon, polyolefins, silicone, polyether block amide etc., with other materials present in the communication element and sensor(s). Blends of different materials may also be used, or layered structures where layers of different materials are sandwiched over each other, e.g. by co-extrusion.

Optionally, the urinary probe 10 may also comprise a hydrophilic coating/surface (preferably of PVP) to make the urinary probe 10 slippery and exhibiting low friction properties. This may make the urinary probe 10 more comfortable when it is inserted through the urethra of a user and/or make it easier to remove the introducer device 4.

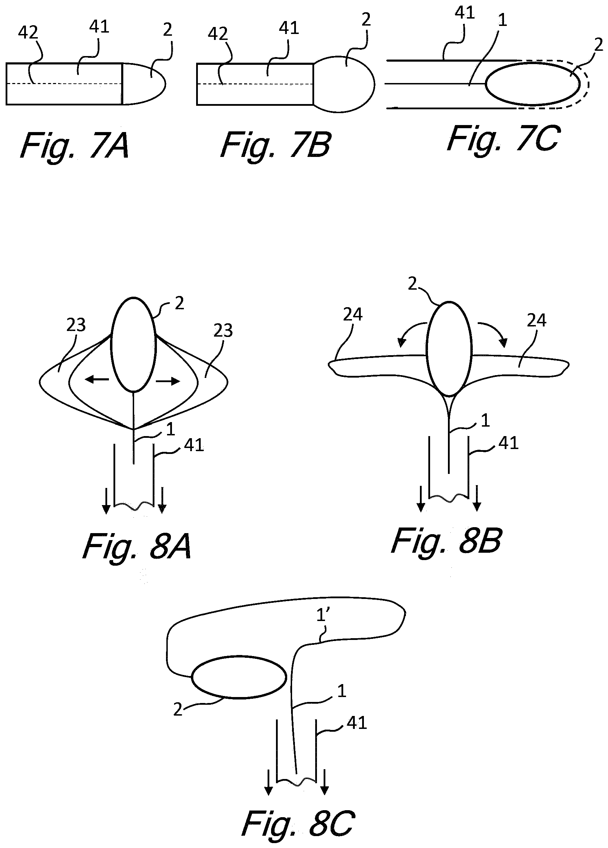

Turning to fig. 2, a cross-sectional view of a urinary probe assembly 20 is shown comprising the urinary probe 10 arranged inside an introducer device 4 comprising a splitable tubular sheath 41. The splitable tubular sheath 41 is preferably arranged to accommodate the whole shaft wire 1, or at least an insertable/proximal part thereof, or at least a substantial part of the shaft wire 1 and/or the insertable part. The splitable tubular sheath 4 may optionally also accommodate at least a part of the, or the entire, sensor unit 2. In the particular embodiment shown in fig. 2, the sensor unit 2 is completely covered by the splitable tubular sheath 41, but this is only one alternative configuration of many, see e.g. also the embodiments depicted in fig. 7A and 7B where the sensor unit 2 is partially covered by the splitable tubular sheath 4.

A part of the shaft wire 1 and the connector 11 may remain outside the introducer device 4 but this is optional, and in some implementations the entire shaft wire 1, and optionally also the connector 11, is arranged inside the introducer device 4.

At least a part of an outer surface of the tubular sheath 41 is provided with a hydrophilic coating/surface, and preferably at least the outer surface of the insertable part of the introducer device 4. In a preferred embodiment, essentially the entire outer surface of the tubular sheath 41 may be provided with the hydrophilic coating/surface. The hydrophilic coating/surface preferably comprises polyvinylpyrrolidone (PVP). However, other hydrophilic polymers are also feasible, and could also be used for the coating/surface, to provide low friction, as is per se known in the art. The hydrophilic coating/surface may be arranged as a hydrophilic coating arranged on a substrate of the tubular sheath 41. However, the hydrophilic coating/surface may alternatively be arranged as an integrated part of the tubular sheath 41, such as an integrated layer, or alternatively, the entire tubular sheath 41 may be made of a hydrophilic material. The hydrophilic coating/surface is preferably arranged to provide low friction when wetted.

The tubular sheath 41 preferably has an inner diameter essentially corresponding to an outer diameter of the shaft wire 1 and/or sensor unit 2. Hereby, the introducer device 4 closely surrounds the shaft wire and/or sensor unit 2, minimizing the outer diameter of the urinary probe arrangement 20.

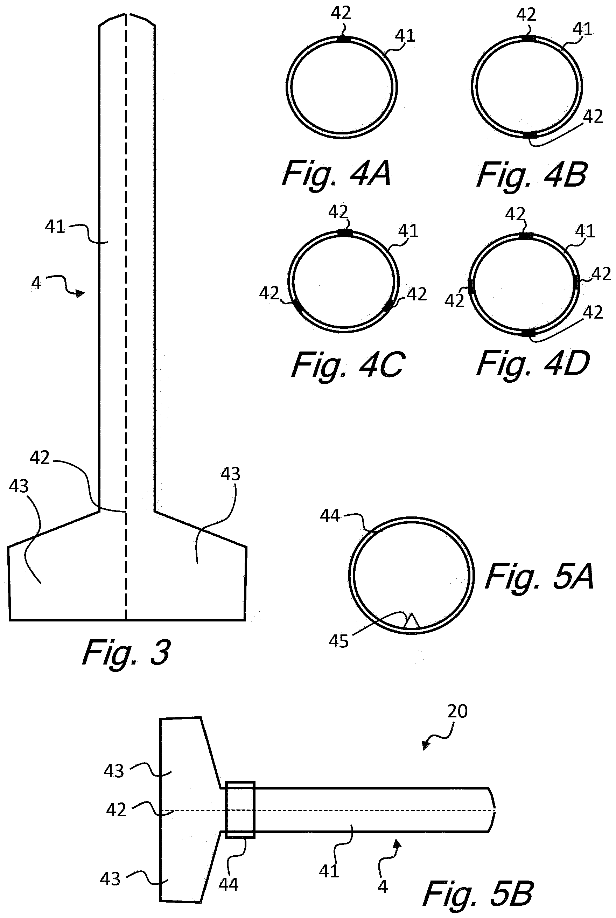

Turning to fig. 3 further details of the introducer device 4 will now be described. In one embodiment, the introducer device 4 comprises a proximal end and a distal end remote from the proximal end, and at least one tear line 42 extending between the proximal and distal ends. The tear line(s) 42 preferably extends along a substantial part of the length of the tubular sheath 41, and preferably over the entire length of the tubular sheath 41, and most preferably along the entire length of the introducer device 4.

The tear line 42 may e.g. be a line forming a weakening in the material, such as a line of perforations, a line made of a different, weaker material, a line made of an interface between two different materials, a line along which the wall thickness is thinner, e.g. by being partly cut through, etc. In an embodiment, the cut through or perforations extend over more than 50% of the wall thickness of the tubular sheath 41, and preferably more than 70%, such as 80-90%. When a force is applied on the introducer device 4, the tear line 42 will preferably be the part of the introducer device 4 that is separated, thereby forming the split. The splitting enables the removal of the introducer device 4 from the shaft wire and sensor unit when it has been inserted into the urethra.

The introducer device 4 may e.g. be produced by extrusion. In such a case, the tear line may e.g. be formed by a cutting device, such as a roller with a cutting edge, arranged close to the extruder, and preferably adjacent or in the vicinity of the outlet nozzle of the extruder. However, a fixed, non-rotatable cutting device may also be used, such as a sharp blade, a cutting tooth, or the like. Hereby, the cutting to form the tear line can be obtained while the extruded material is still warm and soft, which facilitates formation of the tear line, and also provides improved quality of the tear line surfaces and edges.

However, alternatively, the cutting or perforation can be made any time after the extrusion, or the forming of the tubular sheath 41 in other ways.

Alternatively, the introducer device 4 can be produced in other ways, such as by injection molding, etc. It is also possible to produce the introducer device 4 as separate parts which are then connected together. For example, the tubular sheath 41 may be formed by extrusion, and the gripping elements 43 may be formed separately, e.g. by injection molding, and then connected to the tubular sheath 41.

In case a coating is provided on the tubular sheath 41, the coating is preferably made after formation of the tear line(s). However, it is also feasible to form the tear line(s) after the coating step.

In one embodiment only one tear line 42 is provided, as in the illustrative example of fig. 4A. However, more than one tear lines 42 may also be used.

In one embodiment, illustrated in fig. 4B, two tear lines 42 are provided. The two tear lines 42 may extend between the first and second ends, the tear lines 42 being separated from each other in a circumferential direction. For example, the two tear lines 42 may be arranged on opposite sides of the tubular sheath 41, as in the illustrative example. However, other arrangements are also feasible.

By provision of two tear lines 42, the tubular sheath 41 may be split into two separated parts.

More than two tear lines 42 may also be provided. For example, as illustrated in fig. 4C, three tear lines 42 may be provided, and preferably arranged equidistantly separated around the circumference of the tubular sheath 41. Alternatively, four tear lines 42 may be provided, as illustrated in fig. 4D, and preferably arranged equidistantly from each other, in four cardinal directions, such as in a north-south-east-west arrangement.

It is further envisaged that in some implementations two tear lines 42 are arranged close together to enable a narrow strip between the two tear lines 42 to be torn away from the tubular sheath 41. This may be particular advantageous for enabling the tubular sheath 41 to be split with a single hand. For example, a user may use one hand to hold the urinary probe and one hand to open tubular sheath 41. The two tear lines 42 that are arranged close together may e.g. be provided at an angular separation of less than 170°, preferably less than 120°, more preferably less than 90° and most preferably less than 45°.

The tear line(s) 42 may extend generally along the length direction of the tubular sheath 41. However, other paths are also possible, such as helical paths and the like.

In a preferred embodiment, the tear line(s) 42 extend over the entire length of the tubular sheath 41, and/or over the entire length of the introducer device 4.

The introducer device 4 may further comprise at least one gripping element 43 arranged at, or in the vicinity of, a non-insertable distal end of the introducer device 4. The gripping element 43 may e.g. be in the form of handles or flanges protruding out from the tubular sheath 41. In an embodiment, the gripping element 43 may protrude outwardly, at least partially in a radial, lateral direction. In the illustrative embodiment of fig. 3, two gripping elements 43 are provided, preferably arranged separated along the circumference of the tubular sheath 41, and preferably at opposite sides, and protruding in opposite directions. However, only one gripping element 43 may also be used, or more than two gripping elements 43. In one embodiment, the number of gripping elements 43 corresponds to the number of tear lines 42 provided in the tubular sheath 41. In such an embodiment, the gripping elements are preferably arranged between the tear lines 42.

The gripping elements 43 may be in the form of a gripping tab, a gripping ring, etc. The gripping elements 43 may be provided with a texture, such as corrugations, to increase friction when gripped.

The introducer device 4, and in particular the tubular sheath 41, can be made of one or more plastic materials, and in particular polymer and/or thermoplastic materials, such as materials conventionally known for use as catheter shafts and the like. The tubular sheath 41 may e.g. be formed by polyurethane, nylon, polyolefins, silicone, polyether block amide, a thermoplastic elastomer (TPE) etc. Blends of different materials may also be used, or layered structures where layers of different materials are sandwiched over each other, e.g. by co-extrusion.

The tear line(s) 42 may be formed by the use of a separate material. In such embodiments, the tear line(s) 42 may be arranged as a string or stripe of a material which bonds poorly or loosely to a material used in the rest of the tubular sheath. For example, the tubular sheath 41 may comprise polyphenyl ether and the stripes forming the tear lines may comprise polyurethane. However, as discussed in the foregoing, the tear line(s) 42 may alternatively, or additionally, be formed mechanically, by provision of a line of decreased thickness, perforations or the like.

The introducer device 4, and in particular the tubular sheath 41, may also be made of other materials. In one embodiment, the tubular sheath 41 may e.g. be made of a cellulose based material, such as paper. In such embodiments, the tubular sheath 41 may be formed entirely by a cellulose based material, or alternatively, the cellulose based material may be arranged as one layer, and with a further inner and/or outer layer, e.g. made of plastic material, arranged overlying the cellulose based layer.

With reference to fig. 5A and fig. 5B, the urinary probe arrangement 20 may further comprise a guide arrangement 44, such as a ring encircling the introducer device 4, at a position in the vicinity of the non-insertable part of the shaft wire. The guide arrangement 44 is preferably arranged at a non-insertable part of the urinary probe arrangement 20. The guide arrangement may e.g. be connected to the shaft wire, but may alternatively or additionally be connected to the tubular sheath 41, or other parts of the introducer device 4. The guide arrangement 44 may be arranged to maintain the introducer device 4 intact until it has passed the guide arrangement 44, when being pulled to be removed and split. This ensures that the splitting of the introducer device 4 occurs after the ring, at a position outside the urethra. This is of advantage, since splitting of the introducer device 4 may otherwise tend to occur at the urethra orifice, or to some extent inside the urethra. If this happens, it may exert stress on the urethral wall, close to the orifice, which may feel uncomfortable. However, the guiding arrangement 44 is optional and may also be omitted.

The tear line(s) 42 may be weak enough to be easily splitable/severable upon pulling the gripping elements 43 apart using only normal hand or finger force. However, to facilitate separation, and in particular for users having poor dexterity and the like, separation facilitation elements may be provided, to ensure that the separation occurs at the right place(s), and/or to lower the force required to obtain the separation.

To this end, the urinary probe arrangement may, as shown in fig. 5A, comprise a cutting or splitting aid 45 arranged in the vicinity of the non-insertable part of the urinary probe, aiding in severing the introducer device 4. The cutting/splitting aid 45 may e.g. be arranged connected to, or integrated with, the optional guide arrangement 44. The cutting/splitting aid 45 may e.g. comprise a pin, a blade or the like, penetrating into the introducer device, thereby assisting the splitting of the introducer device 4 as it is pulled out passing the cutting aid 44.

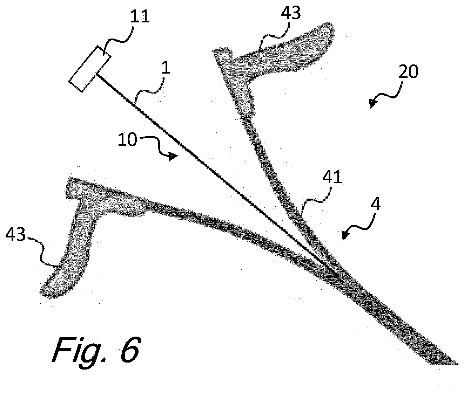

Fig. 6 illustrates how the urinary probe 10 can be released from the introducer device 4 after the tubular sheath 41 has been inserted at least partially in the urethra. The two gripping elements 43 are pulled apart to split the tubular sheath 41 along the one or more tear lines whereby the tubular sheath 41 can be pulled out of the urethra while leaving the urinary probe 10 inside the urethra.

Fig. 7A-C show the details of the tubular sheath 41 in a proximal portion of the of the urinary probe where the sensor unit 2 is arranged. In some implementations, the tubular sheath 41 covers a portion of the sensor unit 2 whereby a portion of the sensor unit 2 protrudes out from the tubular sheath 41, as shown in fig. 7A and fig. 7B. The proximal most part of the tubular sheath 41 may be releasably attached to the sensor unit 2, e.g. fitted tightly around the sensor unit 2, whereby when the tubular sheath 41 is split along the tear line 42, the tubular sheath 41 releases from the sensor unit 2.

It is also envisaged that the tubular sheath 41 covers the entire sensor unit 2 as shown in fig. 7C. For example, at least one tear line may go over the proximal tip of the sensor unit 2 such that when the tubular sheath 41 is pulled out from the urethra sensor unit 2 breaks, and passes through, the tear line going over the proximal tip of the sensor unit, thereby releasing the urinary probe from the tubular sheath 41 such that the tubular sheath 41 can be pulled out around the urinary probe. For example, the tear line (at least at the proximal tip) may become softer and easier to break after coming into contact with urine or after being heated to body temperature, allowing the urinary probe to break through the tear line when the tubular sheath 41 is pulled back. Preferably, the remaining portions of the tear lines are kept intact until the respective portion is outside the urethra (and optionally the guide arrangement 44 from fig. 5A) where it is torn apart.

Additionally or alternatively, a proximal tip of the tubular sheath may be made of a disintegrating material. Hereby, the tip portion formed in the tubular sheath 41, forming the rounded forward end, may be arranged to disintegrate upon contact with water, urine or the like. For example, the proximal tip may be arranged to start to disintegrate upon contact with water, such as when wetting the tubular sheath 41 for activation of the hydrophilic coating/surface. The disintegration process may hereby be slow enough for the urinary probe to be inserted into the urethra with the tubular sheath essentially intact, and thereafter to disintegrate to facilitate removal of the tubular sheath from the urinary probe. Alternatively, or additionally, the proximal end may be arranged to disintegrate, or disintegrate more rapidly, upon contact with urine, so that the proximal end will disintegrate fast as soon as it reaches the bladder.

The tubular sheath 41 may further be provided with a hydrophilic coating/surface also on an inner surface. The hydrophilic coating/surface may be of the same type as the outer hydrophilic coating/surface. Such an inner coating/surface will facilitate the removal of the introducer device, since it will lower the friction between the urinary probe and the tubular sheath 41. Alternatively, another, non-hydrophilic, low friction coating/surface may be used, such as polytetrafluoroethylene (PTFE). However, alternative measures to lower the friction are also feasible, such as providing a lubricating gel or other friction reducing fluids between the introducer device and the urinary probe.

To ensure that the sensor unit 2 remains inside the bladder when the tubular sheath 41 is removed, the urinary probe may comprise one or more retention features that prevents the sensor unit 2 from being pulled out accidentally while removing the tubular sheath 41. Examples of retention features are shown in fig. 8A-C.

Since the sensor unit 2 in general will be of a larger diameter than the shaft wire 1, the sensor unit 2 itself will provide some retention force when the tubular sheath 41 is pulled out of the urethra. In some embodiments, the retention feature comprises one or more retention elements 23 e.g. formed by, or attached to, a section of the sensor unit 2 or shaft wire 1 close to the proximal tip of the urinary probe. The retention elements 23, in a relaxed state, bulges or folds outwardly e.g. due to shape memory. Preferably, at least two expandable retention elements 23 are provided which bulges or folds outwardly, and preferably in opposite directions in the relaxed state.

Alternatively, the retention feature may comprise a plurality of arm sections 24 that form loose ends, each being curved outwardly from the sensor unit 2 or shaft wire 1 in the relaxed state, as illustrated in fig. 8B.

In yet another example, the retention feature is realized by the shaft wire 1, wherein an entire proximal end part 1' of the shaft wire 1 may have a curved disposition in the relaxed state, such as forming a helical shape at the end of the shaft wire, as schematically illustrated in fig. 8C.

The introducer device is preferably arranged to maintain the retention feature in a compressed state when accommodating the shaft wire 1 and sensor unit 2. Hereby, the retention feature is compressed by the introducer device, for easy and simple insertion into the urethra. When the tubular sheath 41 of the introducer device is subsequently removed (or optionally when a tip portion of the tubular sheath disintegrates), the retention feature is allowed to revert to its relaxed state by expanding, curving outwardly or curling, and will then serve to maintain the sensor unit 2 and a portion of the shaft wire 1 in place inside the urinary bladder.

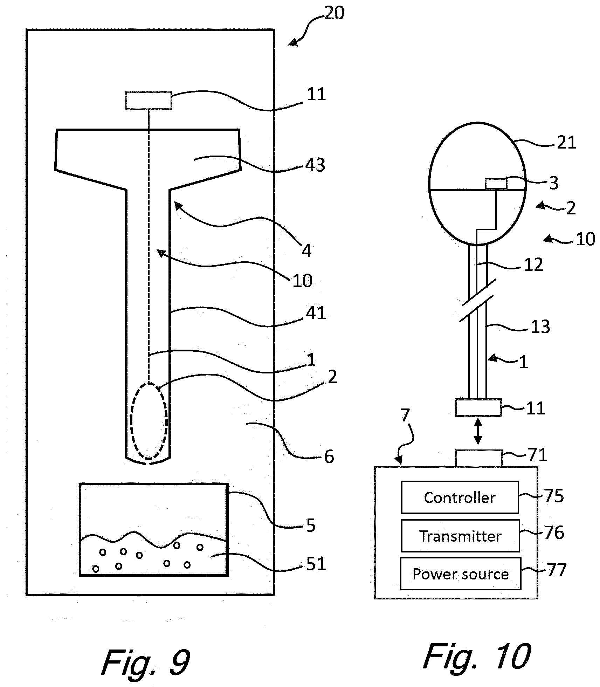

As shown in fig. 9, the urinary probe assembly 20 may further comprise a package 6 accommodating the urinary probe 10 and the introducer device 4. The introducer device 4 is preferably pre-arranged on the urinary probe 10, thereby forming a ready-to-use kit.

The assembly may further comprise a wetting fluid 51 arranged in the package 6. In one embodiment, the wetting fluid 51 is arranged in the package 6 in a separate compartment 5 such as a sachet, , to be released into contact with the hydrophilic coating/surface prior to use. In another embodiment, the wetting fluid 51 is maintained in constant contact with the hydrophilic coating/surface, thereby maintaining the coating/surface in an activated, ready-to-use state.

The package 6 may be of a flexible material, such as foil material. However, the package 6 may also be of a more rigid material, such as rigid plastic material.

The package 6 may, in a closed position, provide a sterile and moisture proof compartment for the introducer device 4 and the urinary probe 10.

Upon use, the urinary probe 10 and introducer device 4 are assembled together, and may e.g. be enclosed in the outer package 6, to maintain the assembly sterile. The wetting fluid (e.g. a liquid, a gas or an aerosol of liquid droplets) 51 may be maintained in constant contact with the hydrophilic surface/coating during storage, thereby providing a urinary catheter assembly which is instantly ready-to-use. In another alternative, the hydrophilic coating/surface of the introducer device 4 is activated by being wetted with a wetting fluid 51, such as water or saline, in preparation for use. The wetting fluid 51 may be provided from an external supply, such as from a tap or the like. However, a supply of wetting fluid 51 may also, as discussed in the foregoing, be integrated in the package 6. For example, a supply of wetting fluid 51 may be arranged in a separate compartment 5 within the package, such as in a sachet, to be released into contact with the hydrophilic coating/surface immediately prior to use.

The connector 11 may also be arranged inside package 6. In some implementations, the connector 11 is insensitive to being wetted with the wetting fluid 51 or can be wiped off before connection to an optional external device. It is also envisaged that the connector 11 is provided in a separate compartment, shielding it from the wetting fluid, whereby the separate compartment is removed prior to connecting the connector 11 to any external device. Only a part of the connector 11 may be sensitive to exposure to the wetting fluid 51 whereby this part can be covered by a sticker, which is removed prior to connecting the connector 11 to an external device.

When the hydrophilic coating/surface is activated, the urinary probe 10 is inserted into the urethra of the user together with the introducer device 4, while maintaining the urinary probe 10 in the tubular sheath 41 of the introducer device 4. This stabilizes the urinary probe 10 during insertion, and makes it more rigid, thereby facilitating insertion and avoiding the risk of kinking and the like.

Once the urinary probe 10 has been inserted, the splitable tubular sheath 41 can be split and the introducer device 4 be removed from the urethra, thereby releasing the urinary probe 10 and leaving it in place in the urethra.

Thus, the combination of the urinary probe 10 and the tubular sheath 41 has increased stability and is easier to push into the urethra than what would have been the case with only the urinary probe 10, without the introducer device 4. This also makes it possible to use a softer, more flexible shaft wire 1, which makes the urinary probe 10 more comfortable during use and which reduces the risk of pain, in particular when used over an extended period of time. For example, it is hereby possible to use urinary probes being so soft and flexible that they could not be inserted into the urethra on their own, without the assistance and stabilization from the introducer device 4. To this end, the shaft wire 1 can be made of softer materials than used conventionally for e.g. urinary catheters, or made very thin. The shaft wire 1 can e.g. be an electrically conductive wire or optical fiber coated with a layer of silicone rubber, latex rubber, polyurethane (PUR) or thermoplastic elastomers (TPE). However, other materials are also feasible. Further, more than one material may be used, and arranged e.g. mixed to a blend or arranged in separate parts, such as in separate layers.

Since the urinary probe 10 can be made much thinner and/or softer than conventional catheters used for cystometric measurements, it is hereby possible to perform comfortable long term cystometric measurements without artificial filling of the bladder. Since the urinary probe 10 is so thin and soft it can, after insertion, be left inside the urethra for many hours, a large portion of a day, one or more days or even one or more weeks allowing the bladder to fill and be emptied naturally (potentially many times) whereby the at least one sensor will measure at least one property (such as the pressure), e.g. when the bladder is emptied. Since the shaft wire 1 is much thinner than a conventional urinary catheter and therefore less obstructive in the urethra, the measurement may also be more accurate in terms of being able to measure a voiding process more closely resembling a natural voiding process, without any device being arranged in the urethra.

Fig. 10 shows schematically how the urinary probe 10 can be used together with an external device 7. The urinary probe 10 (and the introducer device) may be adapted for single time use whereas the external device 7 is adapted for multiple time use. To this end, the external device comprises a connector 71 configured to connect to the connector 11 on urinary probe 10 such that optical and/or electrical signals can be conveyed between the at least one sensor 3 of the sensor unit 2 and the external device 7 via the communication element 12.

In some implementations, the external device 7 comprises a controller 75 for controlling the sensor(s) 3, a transmitter 76 for sending sensor data to a second external device or an external network, and a power source 77 (such as a rechargeable or replaceable battery) for powering the sensor(s) 3, controller 75 and transmitter 76. The external device 7 may further comprise a memory for temporary storage of the measurement data from the sensor(s) 3 and optionally a light and/or sound emitting device for presenting the measurement data or an operational status of the external device 7 and/or urinary probe 10 connected thereto, to a user.

Accordingly, both power and data may be conveyed via the communication element 12 between the at least one sensor 3 and the external device 7. However, it will be appreciated that some components of the external device 7 may be moved to the sensor unit 2 as long as the sensor unit 2 can be made sufficiently small. For example, the transmitter 76 can be integrated in the sensor unit 2 whereby data from the sensor(s) can be transmitted wirelessly to the external device 7 or a second external device or external network thus enabling the external device 7 to only supply power via the communication element 12 with data being conveyed wirelessly.

The sensor(s) 3 may be electrical sensor(s) 3 wherein electrical power and/or electrical data signals are conveyed over the communication element 12 which is realized as an electrical conductor or optionally a plurality of electrical conductors. Optionally, the communication element comprises an optical fiber wherein data is conveyed as optical signals in the optical fiber.

In some implementations, the sensor is a fiber optic sensor, such as a fiber optic pressure sensor and preferably a passive fiber optic pressure sensor which, as such, is known in the art. With a passive fiber optic pressure sensor it is not necessary place any electronics in the sensor unit 2 and no power or electrical signals need to be conveyed to the sensor unit 2, whereby the shaft wire 1 can be made entirely of dielectric materials such as an optical fiber provided with a coating. In this implementation, the connection between connectors 11, 71 can be a purely optical connection with optical light transmitters and receivers being arranged in the external device 7.

As the cystometric measurements are preferably long-term cystometric measurements conducted over one or more hours, days or even weeks the external device 7 is preferably lightweight, compact and adapted for being worn by the user during the measurements. Preferably, the external device is provided with fastening means for holding it against the leg of the user, and preferably against the inside the of a thigh of the user. The fastening means may for example be a strap configured to be wrapped around the leg of the user or an adhesive for temporarily adhering the external device 7 to the leg of the user.

In some implementations, the shaft wire 1, while being thin and soft, exhibits some rigidity. For example, when the urinary probe 10 is held upright it may not be fully self-supporting like a stiff rod but naturally deflect with a bending radius, e.g. due to the sensor unit 3 weighing it down. On the other hand, the shaft wire 1 is not completely without structural integrity like a thread which can be coiled or bundled with essentially no bending radius. Preferably, the external device 7 is therefore placed on the inside of a leg of the user with the connector 71 oriented proximally, facing the urethra. When the shaft wire 1 is inserted into the urethra (using the introducer device) and connected to the connector 71 of the external device 7 the urinary probe 1 may stand on the external device 7 and extend into the bladder or the user via the urethra. The user may even move around, stand up, sit down or lie down while the urinary probe 10 is kept in place at all times by standing on top of the external device 7 and extending through the urethra. Accidental removal of the urinary probe 10 is unlikely since this requires excessive bending of the urinary probe 10 to pull it all the way out of the urinary bladder and urethra.

It is further envisaged that the urinary probe 10 may be held against the user by other means independently of relying on the external device 7. For example, a tape or band-aid, may be arranged close to the urethra and used to hold the urinary probe 10 in place after insertion. The external device 7 may therefore be provided at a more convenient location (e.g. in a pocket or attached to a belt) and connected to the urinary probe 10 wirelessly or using a thin flexible communication wire.

In some implementations, the urinary probe 10 comprises a wireless transmitter (and optionally receiver) arranged in the sensor unit 2 whereby data can be conveyed wirelessly to the external device 7 if it is provided with a wireless receiver and/or transmitter. Optionally, the sensor unit 2 further comprises a power source (such as a battery of fuel cell) whereby it is not necessary to provide external power to the sensor unit 2. Accordingly, in some implementations the urinary probe 10 is powered independently and transmits/receives data wirelessly to the external device whereby the external device 7 is not necessary to connect to the urinary probe 10. The external device 7 and urinary probe 10 can therefore be provided without connectors 11, 21 since it is not necessary to provide optical and/or electrical contact between the two.

In some implementations, the urinary probe 10 comprises a memory configured to store sensor measurement data. Hereby it is not necessary to use either one of a communication element or wireless transmitter since the urinary probe 10 can be inserted into the bladder, perform measurements, store the measurement data in the memory and be removed from the bladder whereby the sensor data in the memory is accessed using e.g. near field communication or by means of a data communication port provided on the urinary probe 10.

Accordingly, it is understood that while additional retention features (as presented in fig. 8A-C) may facilitate retention of the urinary probe 10 inside the bladder, this is not strictly necessary in all implementations since, in some implementations, the urinary probe 10 is kept in place by being retained against the user (e.g. by means of a band-aid or tape or by standing on the external device 7) and extends into the urinary bladder during and/or after removal of the introducer device.

To remove the urinary probe 10, the external device 7 is first dismounted from the patient's leg whereby it is moved away from the urethra, thereby pulling the urinary probe 10 out of the bladder and urethra. Alternatively, the urinary probe 10 is first disconnected from the external device 7 whereby the distal portion of the urinary probe 10 (or the connector 11) is grabbed and pulled away from urethra, thereby pulling the urinary probe 10 out of the bladder and urethra. If the urinary probe is attached to the user by other means (e.g. using a band-aid) these other means are removed and the urinary probe 10 can be pulled out of the urethra.

It is understood that in embodiments wherein the urinary probe 10 comprises a retention feature (see e.g. fig. 8A-C) for keeping at least the sensor unit 2 inside the urinary bladder, a pulling force acting on the urinary probe 10 will stretch, deform and/or compress the retention feature to enable the urinary probe 10 to be pulled out.

Fig. 10 also shows a membrane 21 that at least partially defines a cavity that can be used to protect the sensor(s) 3 from the environment inside the bladder. It is understood that some types of sensors (e.g. salinity or pH sensors) need to be exposed to the inside of the bladder to function. However, other types of sensors such as some types of pressure sensors and temperature sensors are preferably shielded from the urine inside the bladder. For example, the cavity 21 is filled with a gas wherein the pressure sensor is a gas pressure sensor. The fluid pressure inside the urinary bladder will exert a force on the cavity via the membrane 21 which will influence the gas pressure inside the cavity allowing the gas pressure sensor to indirectly measure the pressure inside the bladder.

The person skilled in the art realizes that the present invention by no means is limited to the preferred embodiments described above. On the contrary, many modifications and variations are possible within the scope of the appended claims. For example, various types of sensors may be used in the urinary probe.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

## Claims

1. A urinary probe assembly comprising:
a shaft wire,
a sensor unit, arranged at a proximal end of the shaft wire, wherein the sensor unit comprises at least one sensor configured to measure a property of a urinary bladder and/or a fluid inside the urinary bladder, and
an introducer device including a splitable tubular sheath, wherein the introducer device is arranged to accommodate at least a part of the shaft wire, and wherein at least a part of an outer surface of the tubular sheath is provided with a hydrophilic coating/surface.

2. The urinary probe assembly according to any of the preceding claims, wherein the sensor unit comprises at least one pressure sensor configured to measure a fluid pressure.

3. The urinary probe assembly according to claim 2, wherein the pressure sensor is encased within a fluid filled cavity.

4. The urinary probe assembly according to any of the preceding claims, wherein the sensor unit comprises at least one sensor configured to measure at least one of: temperature, pH and salinity.

5. The urinary probe assembly according to any of the preceding claims, wherein an outer diameter of the shaft wire is less than 3 mm, less than 2 mm, less than 1.5 mm, less than 1 mm, less than 0.75 mm or less than 0.5 mm.

6. The urinary probe assembly according to any of the preceding claims, wherein the introducer device is arranged to accommodate at least an insertable part of the shaft wire.

7. The urinary probe assembly according to any of the preceding claims, wherein the introducer device comprises a first end and a second end remote from the first end, and at least one tear line extending between the first and second ends.

8. The urinary probe assembly according to any of the preceding claims, wherein the introducer device further comprises at least one gripping element arranged at, or in the vicinity of, a non-insertable end of the introducer device.

9. The urinary probe assembly of claim 8, wherein at least two gripping elements are provided, the gripping elements being separated or distributed from each other in a circumferential direction.

10. The urinary probe assembly according to any of the preceding claims, wherein the tubular sheath has a bending stiffness which is greater than the bending stiffness of the shaft wire.

11. The urinary probe assembly according to any of the preceding claims, wherein the sensor unit or shaft wire comprises an expandable retention element, and wherein the introducer device is arranged to maintain the expandable retention element in a compressed state when accommodating the shaft wire.

12. The urinary probe assembly according to any of the preceding claims, wherein the shaft wire comprises a signal transmission element configured to enable communication between a distal end connector of the shaft wire and the at least one sensor of the sensor unit.

13. The urinary probe assembly according to claim 13, wherein the signal transmission element is a conductive wire for electrical communication or an optic waveguide for optical communication.

14. The urinary probe assembly according to any of the preceding claims, wherein the sensor unit comprises a transmitter, configured to wireless transmit measurement acquired by the at least one sensor to an external device.

15. A urinary probe system comprising:
the urinary probe assembly according to claim 12 or claim 13, and
an external device configured to be connected to the distal end connector of the urinary probe.
